# EUROPÄISCHE PATENTSCHRIFT

(11) **EP 3 733 157 B1**
(45) Veröffentlichungstag und Bekanntmachung des Hinweises auf die Patenterteilung: **30.08.2023**
(21) Anmeldenummer: 20170325.3
(22) Anmeldetag: 20.04.2020
(51) Int. Cl.: A61K 8/37, A61K 8/68, A61Q 19/00

(54) **ZUSAMMENSETZUNG ENTHALTEND MINDESTENS EIN CERAMID, MINDESTENS EINE SPHINGOIDBASE UND TRIETHYLCITRAT**
COMPOSITION CONTAINING AT LEAST ONE CERAMIDE, AT LEAST ONE SPHINGOID BASE AND TRIETHYL CITRATE
COMPOSITION CONTENANT AU MOINS UN CÉRAMIDE, AU MOINS UNE BASE SPHINGOÏDE ET DU TRIÉTHYLCITRATE

(30) Priorität: 30.04.2019 EP 19171845
(43) Veröffentlichungstag der Anmeldung: 04.11.2020
(73) Patentinhaber: Evonik Operations GmbH, 45128 Essen (DE)
(72) Erfinder: Maczkiewitz, Ursula, 45219 Essen (DE); Maus, Lisa, 47228 Duisburg (DE); Dietl, Claudia, 45359 Essen (DE); van Logchem, Monica Desiree, 4762 PA Zevenbergen (NL); Mecking, Maria, 46244 Bottrop (DE); Salmina-Petersen, Manuela, 22143 Hamburg (DE); Blasko-Begoihn, Heike, 45359 Essen (DE)
(74) Vertreter: Evonik Patent Association

(56) Entgegenhaltungen:
- JP-A- 2009 167 134
- JP-A- 2011 195 527
- US-A- 5 368 857
- US-A1- 2015 209 405
- DATABASE GNPD [Online] MINTEL; 10. April 2018 (2018-04-10), anonymous: "Sensitive Cream", XP055626967, gefunden im www.gnpd.com Database accession no. 5550201
- DATABASE GNPD [Online] MINTEL; 23. Mai 2018 (2018-05-23), anonymous: "Day Cream SPF 15", XP055627094, gefunden im www.gnpd.com Database accession no. 5685943

## Beschreibung

### Gebiet der Erfindung

Gegenstand der Erfindung ist eine Zusammensetzung enthaltend mindestens ein Ceramid, mindestens eine Sphingoidbase und Triethylcitrat, ein Verfahren zu Herstellung einer Formulierung enthaltend mindestens ein Ceramid und mindestens eine Sphingoidbase sowie die Verwendung von Triethylcitrat zur Stabilisierung einer ceramidhaltigen Formulierung.

### Stand der Technik

Ceramide werden vielfach in kosmetische Formulierungen eingesetzt. Ihre geringe Löslichkeit und große Neigung zu Rekristallisation erschweren jedoch die stabile Einarbeitung in kosmetischen Formulierungen. Insbesondere bei Temperaturen knapp über dem Gefrierpunkt erleiden Formulierungen oft Inhomogenitäten.

Zusätzlich ist eine mikrobielle Stabilisierung von ceramidhaltigen Formulierungen anspruchsvoll, da Konservierungsmittel regelmäßig die physikalische Stabilität von Formulierungen negativ beeinflussen.

Der Stand der Technik adressiert dieses Problem durch verschiedene Darreichungsformen.

So beschreibt die WO1998053797 eingekapselte, wasserunlösliche Wirkstoffe mit amphiphilem Charakter, mit einem Gehalt an Wasser und mindestens einem Tensid aus der Gruppe der Ester von langkettigen Carbonsäuren mit Hydroxylgruppen enthaltenden Carbonsäuren oder deren Salzen und der Ester von langkettigen Carbonsäuren mit Polyalkoholen, wobei Ceramide die wasserunlöslichen Wirkstoffe sein können.

Die WO1999029293 beschreibt Zusammensetzung zur topischen Anwendung, enthaltend eine Kombination einer freien Sphingoidbase und eines Ceramids.

Die JP2008297301 beschreibt Ceramide und Phytostyrol-Derivate in triglyceridhaltigen Öl-Lösungen in flüssiger Form zur Einarbeitung in dermatologische Zubereitungen.

Die US5368857 beschreibt kosmetische Zusammensetzungen, in denen ein Phytosphingosin enthaltendes Ceramid stabil in einer C6-C100-Esterbase suspendiert ist, die als pharmazeutisch verträglicher Träger wirkt, und das Ceramid durch einen Glycerin-C8-C22-Monofettsäureester solubilisiert ist.

Aufgabe der Erfindung war es, Zusammensetzungen enthaltend Ceramide bereitzustellen, die über hervorragende mikrobielle wie auch physikalische Stabilität verfügen.

### Beschreibung der Erfindung

Überraschenderweise wurde gefunden, dass die im Folgenden beschriebenen Zusammensetzungen die der Erfindung gestellte Aufgabe zu lösen vermögen.

Gegenstand der vorliegenden Erfindung sind daher Zusammensetzungen enthaltend A) mindestens ein Ceramid, B) mindestens eine Sphingoidbase und C) Triethylcitrat,
dadurch gekennzeichnet, dass das Gewichtsverhältnis der Komponenten A) zu B) zu C) 2 bis 6 zu 1 bis 2 zu 1 bis 5 beträgt.

Ein weiterer Gegenstand der Erfindung ist ein Verfahren zu Herstellung einer Formulierung enthaltend mindestens ein Ceramid gemäß Anspruch 12 sowie die Verwendung von Triethylcitrat zur physikalischen Stabilisierung einer ceramidhaltigen Formulierung.

Ein Vorteil der vorliegenden Erfindung ist es, dass die erfindungsgemäßen Zusammensetzungen eine erhöhte Lagerstabiliät aufweisen, daher sich langsamer in ihrer Beschaffenheit, insbesondere hinsichtlich ihrer Viskosität, verglichen zu ceramidhaltigen Zusammensetzungen nach dem Stand der Technik über die Zeit verändern.

Noch ein Vorteil der vorliegenden Erfindung ist es, dass die erfindungsgemäßen Zusammensetzungen eine höhere Anzahl von Gefrier-Tauschritten ohne signifikante Viskositätseinbuße vertragen, verglichen zu ceramidhaltigen Zusammensetzungen nach dem Stand der Technik.

Ein Vorteil der erfindungsgemäßen Zusammensetzung ist es, dass die Zusammensetzung in Formulierungen überlegene sensorische Eigenschaften aufweist, die zu einem verbesserten Hautgefühl und/oder Haargefühl führen.

Ein weiterer Vorteil der erfindungsgemäßen Zusammensetzung ist es, dass die Zusammensetzung in Formulierungen in vitro humane follikuläre dermale Papillen-Zellen (HFDPCs) zur Proliferation anregen, was in vivo einer Stimulation des Haarwachstums gleichkommt.

Ein weiterer Vorteil der erfindungsgemäßen Zusammensetzung ist es, dass die Zusammensetzung in Formulierungen verglichen zu den Einzelkomponenten eine verbesserte Verteilbarkeit aufweist. Ein weiterer Vorteil der erfindungsgemäßen Zusammensetzung ist es, dass die Zusammensetzung in Formulierungen verglichen zu den Einzelkomponenten eine verbesserte Absorption aufweist. Ein weiterer Vorteil der erfindungsgemäßen Zusammensetzung ist es, dass die Zusammensetzung in Formulierungen verglichen zu den Einzelkomponenten eine verminderte Öligkeit aufweist.

Ein weiterer Vorteil der erfindungsgemäßen Zusammensetzung ist es, dass die Zusammensetzung in Formulierungen verglichen zu den Einzelkomponenten eine verminderte Wachsigkeit aufweist.

Ein weiterer Vorteil der erfindungsgemäßen Zusammensetzung ist es, dass die Zusammensetzung in Formulierungen verglichen zu den Einzelkomponenten eine verbesserte Gleitfähigkeit aufweist. Ein weiterer Vorteil der erfindungsgemäßen Zusammensetzung ist es, dass die Zusammensetzung in Formulierungen verglichen zu den Einzelkomponenten eine verminderte Klebrigkeit aufweist.

Ein weiterer Vorteil der erfindungsgemäßen Zusammensetzung ist es, dass die Zusammensetzung in Formulierungen verglichen zu den Einzelkomponenten eine verbesserte Seidigkeit/Samtigkeit aufweist.

Unter dem Begriff "Ceramid" im Zusammenhang mit der vorliegenden Erfindung werden acylierte Sphingoidbasen verstanden, wobei die Sphingoidbasen bevorzugt ausgewählt sind aus Sphingosin, Sphinganin, 6-Hydroxysphingosin und Phytosphingosin.

Der "pH-Wert" im Zusammenhang mit der vorliegenden Erfindung ist definiert als der Wert, welcher für die entsprechende Zusammensetzung bei 25 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent.

Bevorzugte erfindungsgemäße Zusammensetzungen sind dadurch gekennzeichnet, dass das Ceramid ausgewählt ist aus der Gruppe Ceramid NP, Ceramid AP, Ceramid EOP, Ceramid NDS , Ceramid ADS, Ceramid EODS, Ceramid NS, Ceramid AS, Ceramid EOS, Ceramid NH, Ceramid AH und Ceramid EOH.

Es ist erfindungsgemäß bevorzugt, dass die erfindungsgemäße Zusammensetzung eine Sphingoidbase ausgewählt aus der Gruppe Sphingosin, Sphinganin, 6-Hydroxysphingosin und Phytosphingosin enthält

Eine besonders bevorzugte erfindungsgemäße Zusammensetzung ist dadurch gekennzeichnet, dass das Ceramid ausgewählt ist aus der Gruppe Ceramid NP und Ceramid AP, und die Sphingoidbase ausgewählt ist aus Phytosphingosin.

Insbesondere ist es bevorzugt, dass die erfindungsgemäße Zusammensetzung als Ceramid Ceramid NP und Ceramid AP enthält, und die Sphingoidbase ausgewählt ist aus Phytosphingosin. In dieser bevorzugten Ausführungsform der erfindungsgemäßen Zusammensetzung ist es bevorzugt, dass Ceramid NP und Ceramid AP in einem Gewichtsverhältnis von 1,5 bis 2,5 zu 1 in der der erfindungsgemäßen Zusammensetzung enthalten ist.

Bevorzugte erfindungsgemäße Zusammensetzungen sind dadurch gekennzeichnet, dass das Gewichtsverhältnis der Komponenten A) zu B) zu C) 2 bis 6 zu 1 bis 2 zu 1 bis 2 beträgt, wobei sich das Gewicht der jeweiligen Komponente auf alle in der Zusammensetzung enthaltenen Ceramide, Sphingoidbasen beziehungsweise Triethylcitrat bezieht.

Bevorzugte erfindungsgemäße Zusammensetzungen stellen Wirkstoffkonzentrate dar, welche einen hohen Aktivgehalt aufweisen; solch eine bevorzugte erfindungsgemäße Zusammensetzung ist dadurch gekennzeichnet, dass Komponenten A) und B) in Summe in einer Menge von 1,0 Gew.-% bis 9,0 Gew.-%, bevorzugt von 1,5 Gew.-% bis 5,0 Gew.-%, besonders bevorzugt von 2,0 Gew.-% bis 3,0 Gew.-%, enthalten sind, wobei sich die Gewichtsprozente auf die Gesamtzusammensetzung beziehen.

Aus diesen Wirkstoffkonzentraten lassen sich durch Abmischung Formulierungen, insbesondere kosmetische Formulierungen, herstellen, bei denen die Wirkstoffkonzentration im Vergleich zu den vorgenannten Konzentraten erniedrigt ist; solch eine alternative, bevorzugte erfindungsgemäße Zusammensetzung ist dadurch gekennzeichnet, dass Komponenten A) und B) in Summe in einer Menge von 0,02 Gew.-% bis 0,60 Gew.-%, bevorzugt von 0,03 Gew.-% bis 0,40 Gew.-%, besonders bevorzugt von 0,05 Gew.-% bis 0,30 Gew.-%, enthalten sind, wobei sich die Gewichtsprozente auf die Gesamtzusammensetzung beziehen.

Solche alternativen, bevorzugten erfindungsgemäßen Zusammensetzungen sind insbesondere Formulierungen, besonders bevorzugt kosmetische.

Die erfindungsgemäßen Formulierungen können des Weiteren mindestens eine zusätzliche Komponente enthalten, ausgewählt aus der Gruppe der
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Geruchsabsorber,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Es ist erfindungsgemäß bevorzugt, dass die erfindungsgemäße Zusammensetzung der vorliegenden Erfindung enthält D) Cholesterol, insbesondere als erfindungsgemäßes Wirkstoffkonzentrate, bevorzugt in einer Menge von 0,1 Gew.-% bis 3,0 Gew.-%, bevorzugt von 0,3 Gew.-% bis 2,0 Gew.-%, besonders bevorzugt von 0,4 Gew.-% bis 1,0 Gew.-%, wobei sich die Gewichtsprozente auf die Gesamtzusammensetzung beziehen.

Erfindungsgemäß bevorzugte Zusammensetzungen der vorliegenden Erfindung sind Emulsionen, insbesondere Öl-in-Wasser-Emulsionen.

Daher ist es erfindungsgemäß bevorzugt, dass die erfindungsgemäße Zusammensetzung der vorliegenden Erfindung enthält E) mindestens einen Emulgator, insbesondere als erfindungsgemäßes Wirkstoffkonzentrate in einer Menge von 0,1 Gew.-% bis 3,0 Gew.-%, bevorzugt von 0,3 Gew.-% bis 2,0 Gew.-%, besonders bevorzugt von 0,4 Gew.-% bis 1,0 Gew.-%, wobei sich die Gewichtsprozente auf die Gesamtzusammensetzung beziehen..

Unter dem Begriff "Emulgator" im Zusammenhang mit der vorliegenden Erfindung werden organische Substanzen mit grenzflächenaktiven Eigenschaften verstanden, die die Fähigkeit besitzen können, die Oberflächenspannung von Wasser bei 20°C und bei einer Konzentration von 0,5 Gew.-% bezogen auf die Gesamtzusammensetzung auf unter 45 mN/m zu verringern, und für die kein pH-Wert zwischen 2 und 12 existiert, bei dem bei 20 °C mindestens 50 mol-% der Moleküle ionisch geladen vorliegen. Diese Substanzen liegen also bei allen pH-Werten zwischen 2 und 12 größtenteils insgesamt nach Außen ungeladen vor.

Die Oberflächenspannung wird hier durch die Ringmethode nach DuNoüy bei 20°C bestimmt. Bevorzugt enthaltene Emulgatoren sind ausgewählt aus der Gruppe Acyl-Lactylate (insbesondere Sodium Lauroyl Lactylate), ethoxylierte Fettalkohole (insbesondere Ceteareth-25), ethoxylierte Fettsäuren (insbesondere PEG_100 Stearate), Polyglycerinester (insbesondere Polyglyceryl-3 Methylglucose Distearate, Polyglyceryl-6 Stearate; Polyglyceryl-6 Behenate), Glycerinester, (insbesondere Glyceryl Stearate Citrate), ethoxylierte Sorbitanester und Lecithin.

Unter dem Begriff "Acyl-Lactylat" im Zusammenhang mit der vorliegenden Erfindung werden salzförmige Umsetzungsprodukte von Fettsäure mit Milchsäure und/oder Polymilchsäure verstanden.

Eine besonders bevorzugte erfindungsgemäße Zusammensetzung ist dadurch gekennzeichnet, dass sie einen pH-Wert im Bereich von 4,0 bis 8,0, bevorzugt von 4,5 bis 7,4 besonders bevorzugt von 5,5 bis 6,9, alternativ besonders bevorzugt von 4,0 bis 5,4 , aufweist.

Ein weiterer Gegenstand der vorliegenden Erfindung ist ein Verfahren zur Herstellung einer Formulierung enthaltend A) mindestens ein Ceramid und B) mindestens eine Sphingoidbase, umfassend die Verfahrensschritte:
a) Bereitstellen des mindesten eines Ceramides und Bereitstellen von C) Triethylcitrat und Bereitstellen der mindestens einen Sphingoidbase;
b) Vermengen der Komponenten A) und C) und B) zusammen mit Wasser zu einer Lösung der Komponente A).

Die mit dem erfindungsgemäßen Verfahren hergestellten Formulierungen sind bevorzugt die oben beschriebenen erfindungsgemäßen Zusammensetzungen, wobei bevorzugte erfindungsgemäße Zusammensetzungen ebenfalls bevorzugt mit dem erfindungsgemäßen Verfahren hergestellt werden.

Somit wird beispielsweise in dem erfindungsgemäßen Verfahren das Gewichtsverhältnis der Komponenten A) zu B) zu C) bevorzugt in einem Gewichtsverhältnis von 2 bis 6 zu 1 bis 2 zu 1 bis 5, bevorzugt von 2 bis 6 zu 1 bis 2 zu 1 bis 2, eingesetzt, wobei sich das Gewicht der jeweiligen Komponente auf alle im Verfahren eingesetzte Ceramide, Sphingoidbasen beziehungsweise Triethylcitrat bezieht.

Die mit dem erfindungsgemäßen Verfahren hergestellten Formulierungen sind insbesondere physikalisch stabile Formulierungen. Unter dem Begriff "physikalisch stabile Formulierungen enthaltend mindestens ein Ceramid" im Zusammenhang mit der vorliegenden Erfindung werden insbesondere Formulierungen verstanden, die insbesondere keine Auskristallisation der Ceramide und der Sphingoidbase zeigen, sowie keine Separation oder Inhomogenität nach sechsmehrmonatiger Lagerung bei 25 °C.

Es ist erfindungsgemäß vorteilhaft und daher bevorzugt, wenn das erfindungsgemäße Verfahren dadurch gekennzeichnet ist, dass Verfahrensschritt b) mindesten teilweise in einem Temperaturbereich von 70 °C bis 110 °C, bevorzugt von 75 °C bis 100 °C, besonders bevorzugt von 80 °C bis 90 °C, durchgeführt wird.

Insbesondere erfindungsgemäß bevorzugt ist es, wenn das erfindungsgemäße Verfahren dadurch gekennzeichnet ist, dass in Verfahrensschritt b) Komponenten A), bevorzugt und B), mit Wasser zu einer homogenen Lösung in einem Temperaturbereich von 70 °C bis 110°C, bevorzugt von 75 °C bis 100 °C, besonders bevorzugt von 80 °C bis 90 °C, vermengt werden und Komponente C) in einem Temperaturbereich von 5 °C bis 50 °C, bevorzugt von 10 °C bis 40 °C, besonders bevorzugt von 15 °C bis 30 °C, mit der homogenen Lösung vermengt wird.

Ein bevorzugtes erfindungsgemäßes Verfahren ist dadurch gekennzeichnet, dass in Verfahrensschritt b) die Lösung einen pH-Bereich von 4,0 bis 8,0, bevorzugt von 4,5 bis 7,4 besonders bevorzugt von 5,5 bis 6,9, alternativ besonders bevorzugt von 4,0 bis 5,4, aufweist.

Ein besonders bevorzugtes erfindungsgemäßes Verfahren ist dadurch gekennzeichnet, dass in Verfahrensschritt b) mindestens ein Emulgator enthalten ist.

Dieser wird erfindungsgemäß bevorzugt in Verfahrensschritt b) in einem Temperaturbereich von 70 °C bis 110 °C, bevorzugt von 75 °C bis 100 °C, besonders bevorzugt von 80 °C bis 90 °C, mit den andere Komponenten vermengt.

Bevorzugt eingesetzte Emulgatoren sind ausgewählt aus der Gruppe Sodium Lauroyl Lactylate, ethoxylierte Fettalkohole (insbesondere Ceteareth-25), ethoxylierte Fettsäuren (insbesondere PEG_100 Stearate), Polyglycerinester (insbesondere Polyglyceryl-3 Methylglucose Distearate, Polyglyceryl-6 Stearate; Polyglyceryl-6 Behenate), Glycerinester, (insbesondere Glyceryl Stearate Citrate), ethoxylierte Sorbitanester und Lecithin.

Das bevorzugte Gewichtsverhältnis der Komponenten A) und B) in Summe zu dem Emulgator liegt im Verfahrensschritt b) bei von 1 zu 1 bis 1 zu 20, bevorzugt von 1:2 bis 1:10, wobei sich die Gewichte auf alle enthaltenen Ceramide, Sphingoidbasen und Emulgatoren beziehen.

Die weiteren bevorzugten Gewichtsverhältnisse der Einzelkomponenten zueinander im erfindungsgemäßen Verfahren entsprechen den bevorzugten der erfindungsgemäßen Zusammensetzungen.

Analoges gilt für die bevorzugten Komponenten A), B) und C) sowie Emulgator.

Es wurde völlig überraschend gefunden, dass sich Triethylcitrat vorteilhaft auf die Stabilisierung von ceramidhaltigen Formulierungen auswirkt; daher ist ein weiterer Gegenstand der vorliegenden Erfindung die Verwendung von Triethylcitrat zur physikalischen Stabilisierung einer ceramidhaltigen Formulierung, insbesondere hinsichtlich Homogenität, sowie bevorzugt und zur mikrobiellen Stabilisierung einer ceramidhaltigen Formulierung, insbesondere in Bezug auf mindestens einen ausgewählt aus *S*. *aureaus*, *P. aeruginosa*, *E. coli,* C. *albicans* und *A*. *brasiliensis.*

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Mikrobielle Stabilität

Ceramidhaltige Formulierungen wurden unter Verwendung verschiedener Konservierungsmittelkombinationen hergestellt.

Die Zusammensetzung der ceramidhaltigen Formulierung findet sich in Tabelle 1 (die Konzentrationsangaben sind Gewichts-%):

**Tabelle 1: ceramidhaltige Formulierungen**

| Phase | Inhaltsstoff | w/w% |
|---|---|---|
| A | Sodium Lauroyl Lactylate | 10.0 |
| | Ceramide NP | 1.12 |
| | Ceramide AP | 0.53 |
| | Phytosphingosine | 0.54 |
| | Cholesterol | 0.50 |
| B | Wasser | ad 100.0 |
| | Carbomer | 0.30 |
| | Natriumhydroxid (10% in Wasser) | ad pH 6 |
| C | Xanthan Gum | 0.30 |
| D | Konservierungsmittel | x |

### Die Herstellung erfolgte folgendermaßen:

Phase A und B wurden auf 85 °C erwärmt. Dann wurde Phase A langsam unter Rühren mit einem Ystrahl-Rührer zu Phase B gegeben. Anschließend wurde auf 70 °C abgekühlt, Phase C zugegeben und solange mit dem Ystrahl homogenisiert, bis die Formulierung homogen war. Unter Rühren wurde anschließend auf 25 °C abgekühlt.

Zu dieser Formulierung wurden als letzter Schritt die verschiedenen Konservierungsmittel zugegeben und homogen verrührt.

Die so erhaltenen Formulierungen wurden einem Konservierungsbelastungstest (KBT) unterzogen. Dieser Test wurde nach der entsprechenden Vorschrift aus der European Pharmacopoeia 7.0, 5.1.3 *Efficacy of antimicrobial preservation*, durchgeführt. Erfüllte der Test bei allen Organismen nicht mindestens Kriterium B, so galt der KBT als "nicht bestanden".

Getestet wurden die Mikroorganismen *S*. *aureaus*, *P. aeruginosa*, *E. coli*, *C. albicans* und *A. brasiliensis:*
Die folgende Tabelle fasst die Ergebnisse zusammen.

**Tabelle 2: Ergebnisse des Konservierungsbelastungstest**

| x% Konservierungsmittel | erfindungsgemäß | enthält | Ergebnis KBT |
|---|---|---|---|
| 1% Euxyl PE 9010 + 0.2% Sensiva SC 50 | nein | Phenoxyethanol, Ethylhexylglycerin | nicht bestanden |
| 1.5% Sensiva PA 40 | nein | Phenylpropanol, Propanediol, Caprylyl Glycol, Tocopherol | nicht bestanden |
| 2% Sensiva SC 10 | nein | Caprylyl Glycol, Ethylhexylglycerin | nicht bestanden |
| 1% Euxyl K 900 | nein | Benzylalcohol, Ethylhexylglycerin, Tocopherol | nicht bestanden |
| 3% dermosoft OMP | nein | Methylpropanediol, Caprylyl Glycol, Phenylpropanol | nicht bestanden |
| 1.5% Verstatil SL + 3% dermosoft OM | nein | Sodium Levulinate, Potassium Sorbate, Methylpropanediol, Caprylyl Glycol | nicht bestanden |
| 3% dermosoft OMP + 2% dermofeel tec eco | ja | Methylpropanediol, Caprylyl Glycol, Phenylpropanol, Triethylcitrat | bestanden |
| 2% Verstatil SL + 2% dermofeel tec eco | ja | Sodium Levulinate, Potassium Sorbate, **Triethylcitrat** | bestanden |
| 1.5% Verstatil TBO | ja | **Triethylcitrat**, Caprylyl Glycol, Benzoic Acid | bestanden |

Überraschenderweise wurde gefunden, dass Konservierungsmittelkombinationen, die Triethylcitrat enthalten, die Anforderungen des Konservierungsbelastungstest erfüllen, während Konservierungsmittelkombinationen ohne Triethylcitrat dies nicht schaffen.

### Beispiel 2: Physikalische Stabilität

Die ceramidhaltigen Formulierungen aus Beispiel 1 mit den verschiedenen Konservierungsmitteln wurden zusätzlich auf Rekristallisation der Ceramide geprüft. Dies geschah mit einem Lichtmikroskop vom Typ Olympus IX83. In der Tabelle 3 werden die Ergebnisse der Mikroskopuntersuchung zusammengefasst.

**Tabelle 3: Ergebnisse der Mikroskopuntersuchungen**

| Konservierungsmittel | erfindungsgemäß | Zusammensetzung | Ergebnis der Mikroskopuntersuchung |
|---|---|---|---|
| 1% Euxyl PE 9010 + 0.2% Sensiva SC 50 | nein | Phenoxyethanol, Ethylhexylglycerin | Rekristallisation der Ceramide |
| 1.5% Sensiva PA 40 | nein | Phenylpropanol, Propanediol, Caprylyl Glycol, Tocopherol | Rekristallisation der Ceramide |
| 2% Sensiva SC 10 | nein | Caprylyl Glycol, Ethylhexylglycerin | Rekristallisation der Ceramide |
| 1% Euxyl K 900 | nein | Benzylalcohol, Ethylhexylglycerin, Tocopherol | Rekristallisation der Ceramide |
| 3% dermosoft OMP + 2% dermofeel tec eco | ja | Methylpropanediol, Caprylyl Glycol, Phenylpropanol, **Triethylcitrat** | keine Rekristallisation |
| 2% Verstatil SL + 2% dermofeel tec eco | ja | Sodium Levulinate, Potassium Sorbate, Triethylcitrat | keine Rekristallisation |
| 1.5% Verstatil TBO | ja | **Triethylcitrat**, Caprylyl Glycol, Benzoic Acid | keine Rekristallisation |

Überraschenderweise wurde auch hier gefunden, dass das Triethylcitrat die Rekristallisation von Ceramiden verhindert, während Konservierungsmittel, die kein Triethylcitrat enthalten, dazu nicht in der Lage waren. Ohne Triethylcitrat zeigten die ceramidhaltigen Formulierungen eine deutliche Rekristallisation der Ceramide.

## Patentansprüche

1. Zusammensetzung enthaltend
A) mindestens ein Ceramid,
B) mindestens eine Sphingoidbase und
C) Triethylcitrat,
**dadurch gekennzeichnet, dass** das Gewichtsverhältnis der Komponenten A) zu B) zu C) 2 bis 6 zu 1 bis 2 zu 1 bis 5 beträgt.

2. Zusammensetzung nach Anspruch 1, **dadurch gekennzeichnet, dass**
das Ceramid ausgewählt ist aus der Gruppe Ceramid NP, Ceramid AP, Ceramid EOP, Ceramid NDS, Ceramid ADS, Ceramid EODS, Ceramid NS, Ceramid AS, Ceramid EOS, Ceramid NH, Ceramid AH und Ceramid EOH.

3. Zusammensetzung nach Anspruch 1 oder 2, **dadurch gekennzeichnet, dass**
die Sphingoidbase ausgewählt ist aus der Gruppe Sphingosin, Sphinganin, 6-Hydroxysphingosin und Phytosphingosin.

4. Zusammensetzung nach mindestens einem der vorherigen Ansprüche, **dadurch gekennzeichnet, dass**
das Ceramid ausgewählt ist aus der Gruppe Ceramid NP und Ceramid AP, und
die Sphingoidbase ausgewählt ist aus Phytosphingosin.

5. Zusammensetzung nach mindestens einem der vorherigen Ansprüche, **dadurch gekennzeichnet, dass**
als Ceramid Ceramid NP und Ceramid AP enthalten ist, und
die Sphingoidbase ausgewählt ist aus Phytosphingosin.

6. Zusammensetzung nach mindestens einem der vorherigen Ansprüche, **dadurch**
**gekennzeichnet, dass** das Gewichtsverhältnis der Komponenten A) zu B) zu C) 2 bis 6 zu 1 bis 2 zu 1 bis 2 beträgt.

7. Zusammensetzung nach Anspruch 5 oder 6, **dadurch gekennzeichnet, dass**
Ceramid NP und Ceramid AP in einem Gewichtsverhältnis von 1,5 bis 2,5 zu 1 enthalten ist.

8. Zusammensetzung nach mindestens einem der vorherigen Ansprüche, **dadurch**
**gekennzeichnet, dass** Komponenten A) und B) in Summe in einer Menge von 1,0 Gew.-% bis 9,0 Gew.-%, bevorzugt von 1,5 Gew.-% bis 5,0 Gew.-%, besonders bevorzugt von 2,0 Gew.-% bis 3,0 Gew.-%, enthalten sind, wobei sich die Gewichtsprozente auf die Gesamtzusammensetzung beziehen.

9. Zusammensetzung nach mindestens einem der Ansprüche 1 bis 7, **dadurch gekennzeichnet, dass** Komponenten A) und B) in Summe in einer Menge von 0,02 Gew.-% bis 0,60 Gew.-%, bevorzugt von 0,03 Gew.-% bis 0,40 Gew.-%, besonders bevorzugt von 0,05 Gew.-% bis 0,30 Gew.-%, enthalten sind, wobei sich die Gewichtsprozente auf die Gesamtzusammensetzung beziehen.

10. Zusammensetzung nach mindestens einem der vorherigen Ansprüche, **dadurch gekennzeichnet, dass** sie enthält
D) Cholesterol,
bevorzugt in einer Menge von 0,1 Gew.-% bis 3,0 Gew.-%, bevorzugt von 0,3 Gew.-% bis 2,0 Gew.-%, besonders bevorzugt von 0,4 Gew.-% bis 1,0 Gew.-%, wobei sich die Gewichtsprozente auf die Gesamtzusammensetzung beziehen.

11. Zusammensetzung nach mindestens einem der vorherigen Ansprüche, **dadurch gekennzeichnet, dass** sie einen pH-Wert im Bereich 4,0 bis 8,0, bevorzugt von 4,5 bis 7,4 besonders bevorzugt von 5,5 bis 6,9, alternativ besonders bevorzugt von 4,0 bis 5,4, aufweist.

12. Verfahren zu Herstellung einer Formulierung enthaltend A) mindestens ein Ceramid und B) mindestens eine Sphingoidbase umfassend die Verfahrensschritte:
a) Bereitstellen des mindestens einen Ceramides und Bereitstellen von C) Triethylcitrat und Bereitstellen der mindestens einen Sphingoidbase;
b) Vermengen der Komponenten A) und C) und B), zusammen mit Wasser zu einer Lösung der Komponente A).

13. Verfahren nach Anspruch 12, **dadurch gekennzeichnet, dass** Verfahrensschritt b) mindestens teilweise in einem Temperaturbereich von 70 °C bis 110 °C, bevorzugt von 75 °C bis 100 °C, besonders bevorzugt von 80 °C bis 90 °C, durchgeführt wird.

14. Verfahren nach Anspruch 12 oder 13, **dadurch gekennzeichnet, dass** in Verfahrensschritt b) die Lösung einen pH-Bereich von 4,0 bis 8,0, bevorzugt von 4,5 bis 7,4 besonders bevorzugt von 5,5 bis 6,9, alternativ besonders bevorzugt von 4,0 bis 5,4, aufweist.

15. Verwendung von Triethylcitrat zur physikalischen Stabilisierung einer ceramidhaltigen Formulierung, insbesondere hinsichtlich Homogenität.

16. Verwendung von Triethylcitrat gemäß Anspruch 15 und zur mikrobiellen Stabilisierung der ceramidhaltigen Formulierung, insbesondere in Bezug auf mindestens einen ausgewählt aus *S*. *aureaus*, *P. aeruginosa, E. coli*, *C. albicans* und *A*. *brasiliensis.*

## Claims

1. Composition comprising
A) at least one ceramide,
B) at least one sphingoid base and
C) triethyl citrate,
**characterized in that** the ratio by weight of components A) to B) to C) is from 2:6:1 to 2:1:5.

2. Composition according to Claim 1, **characterized in that**
the ceramide is selected from the group comprising ceramide NP, ceramide AP, ceramide EOP, ceramide NDS, ceramide ADS, ceramide EODS, ceramide NS, ceramide AS, ceramide EOS, ceramide NH, ceramide AH and ceramide EOH.

3. Composition according to Claim 1 or 2, **characterized in that**
the sphingoid base is selected from the group comprising sphingosine, sphinganine, 6-hydroxysphingosine and phytosphingosine.

4. Composition according to at least one of the preceding claims, **characterized in that**
the ceramide is selected from the group comprising ceramide NP and ceramide AP, and the sphingoid base is selected from phytosphingosine.

5. Composition according to at least one of the preceding claims, **characterized in that**
the ceramide present is ceramide NP and ceramide AP, and the sphingoid base is selected from phytosphingosine.

6. Composition according to at least one of the preceding claims, **characterized in that** the ratio by weight of components A) to B) to C) is from 2:6:1 to 2:1:2.

7. Composition according to Claim 5 or 6, **characterized in that**
ceramide NP and ceramide AP is present in a ratio by weight from 1.5 to 2.5:1.

8. Composition according to at least one of the preceding claims, **characterized in that** components A) and B) are present in total in an amount of 1.0% by weight to 9.0% by weight, preferably 1.5% by weight to 5.0% by weight, particularly preferably 2.0% by weight to 3.0% by weight, wherein the percentages by weight refer to the total composition.

9. Composition according to at least one of Claims 1 to 7, **characterized in that** components A) and B) are present in total in an amount of 0.02% by weight to 0.60% by weight, preferably 0.03% by weight to 0.40% by weight, particularly preferably 0.05% by weight to 0.30% by weight, wherein the percentages by weight refer to the total composition.

10. Composition according to at least one of the preceding claims, **characterized in that** said composition comprises
D) cholesterol,
preferably in an amount of 0.1% by weight to 3.0% by weight, preferably 0.3% by weight to 2.0% by weight, particularly preferably 0.4% by weight to 1.0% by weight, wherein the percentages by weight refer to the total composition.

11. Composition according to at least one of the preceding claims, **characterized in that** said composition has a pH in the range of 4.0 to 8.0, preferably 4.5 to 7.4, particularly preferably 5.5 to 6.9, alternatively particularly preferably 4.0 to 5.4.

12. Process for producing a formulation comprising A) at least one ceramide and B) at least one sphingoid base, comprising the process steps of:
a) providing the at least one ceramide and providing C) triethyl citrate and providing the at least one sphingoid base;
b) mixing components A) and C) and B), together with water, to give a solution of component A).

13. Process according to Claim 12, **characterized in that** process step b) is carried out at least partially in a temperature range of 70°C to 110°C, preferably 75°C to 100°C, particularly preferably 80°C to 90°C.

14. Process according to Claim 12 or 13, **characterized in that** in process step b) the solution has a pH range of 4.0 to 8.0, preferably 4.5 to 7.4, particularly preferably 5.5 to 6.9, alternatively particularly preferably 4.0 to 5.4.

15. Use of triethyl citrate for physical stabilization of a ceramide-containing formulation, in particular with regard to homogeneity.

16. Use of triethyl citrate according to Claim 15 and for microbial stabilization of the ceramide-containing formulation, in particular with respect to at least one selected from *S. aureaus*, *P. aeruginosa*, *E. coli*, *C*. *albicans* and *A*. *brasiliensis.*

## Revendications

1. Composition contenant
A) au moins un céramide,
B) au moins une base sphingoïde et
C) du citrate de triéthyle,
**caractérisée en ce que** la proportion en poids des composants A):B):C) est de 2 à 6:1 à 2:1 à 5.

2. Composition selon la revendication 1, **caractérisée en ce que** le céramide est choisi dans le groupe céramide NP, céramide AP, céramide EOP, céramide NDS, céramide ADS, céramide EODS, céramide NS, céramide AS, céramide EOS, céramide NH, céramide AH et céramide EOH.

3. Composition selon la revendication 1 ou 2, **caractérisée en ce que** la base sphingoïde est choisie dans le groupe sphingosine, sphinganine, 6-hydroxysphingosine et phytosphingosine.

4. Composition selon au moins l'une des revendications précédentes, **caractérisée en ce que** le céramide est choisi dans le groupe céramide NP et céramide AP, et la base sphingoïde est choisie parmi la phytosphingosine.

5. Composition selon au moins l'une des revendications précédentes, **caractérisée en ce qu'**elle contient comme céramide le céramide NP et le céramide AP, et la base sphingoïde est choisie parmi la phytosphingosine.

6. Composition selon au moins l'une des revendications précédentes, **caractérisée en ce que** la proportion en poids des composants A) :B) :C) est de 2 à 6:1 à 2:1 à 2.

7. Composition selon la revendication 5 ou 6, **caractérisée en ce qu'**elle contient le céramide NP et le céramide AP selon une proportion pondérale de 1,5 à 2,5:1.

8. Composition selon au moins l'une des revendications précédentes, **caractérisée en ce qu'**elle contient les composants A) et B) en tout en une quantité de 1,0 % en poids à 9,0 % en poids, de préférence de 1,5 % en poids à 5,0 % en poids, d'une manière particulièrement préférée de 2,0 % en poids à 3,0 % en poids, les pourcentages en poids étant rapportés à la composition totale.

9. Composition selon au moins l'une des revendications 1 à 7, **caractérisée en ce qu'**elle contient les composants A) et B) en tout en une quantité de 0,02 % en poids à 0,60 % en poids, de préférence de 0,03 % en poids à 0,40 % en poids, d'une manière particulièrement préférée de 0,05 % en poids à 0,30 % en poids, les pourcentages en poids étant rapportés à la composition totale.

10. Composition selon au moins l'une des revendications précédentes, **caractérisée en ce qu'**elle contient
D) du cholestérol, de préférence en une quantité de 0,1 % en poids à 3,0 % en poids, de préférence de 0,3 % en poids à 2,0 % en poids, d'une manière particulièrement préférée de 0,4 % en poids à 1,0 % en poids, les pourcentages en poids étant rapportés à la composition totale.

11. Composition selon au moins l'une des revendications précédentes, **caractérisée en ce qu'**elle présente un pH dans la plage de 4,0 à 8,0, de préférence de 4,5 à 7,4, d'une manière particulièrement préférée de 5,5 à 6,9, ou encore d'une manière particulièrement préférée de 4,0 à 5,4.

12. Procédé de fabrication d'une formulation contenant A) au moins un céramide et B) au moins une base sphingoïde, comprenant les étapes suivantes :
a) fourniture de l'au moins un céramide et fourniture de C) du citrate de triéthyle et fourniture de l'au moins une base sphingoïde ;
b) mélange des composants A) et C) et B) conjointement avec de l'eau pour obtenir une solution du composant A).

13. Procédé selon la revendication 12, **caractérisé en ce que** l'étape b) est mise en œuvre au moins partiellement dans la plage de températures de 70 °C à 110 °C, de préférence de 75 °C à 100 °C, d'une manière particulièrement préférée de 80 °C à 90 °C.

14. Procédé selon la revendication 12 ou 13, **caractérisé en ce que** dans l'étape b), la solution présente une plage de pH de 4,0 à 8,0, de préférence de 4,5 à 7,4, d'une manière particulièrement préférée de 5,5 à 6,9, ou encore d'une manière particulièrement préférée de 4,0 à 5,4.

15. Utilisation de citrate de triéthyle pour la stabilisation physique d'une formulation contenant un céramide, en particulier vis-à-vis de l'homogénéité.

16. Utilisation de citrate de triéthyle selon la revendication 15 et pour la stabilisation microbienne de la formulation contenant un céramide, en particulier en liaison avec au moins l'un choisi parmi *S. aureaus*, *P. aeruginosa*, *E. coli*, *C. albicans* et *A. brasiliensis.*
